# FASCICULE DE BREVET EUROPEEN

(11) **EP 3 223 696 B1**
(45) Date de publication et mention de la délivrance du brevet: **10.01.2024**
(21) Numéro de dépôt: 15820268.9
(22) Date de dépôt: 24.11.2015
(51) Int. Cl.: A61B 5/318, A61B 5/389, A61B 5/00, A61H 23/02

(54) **DISPOSITIF DE STIMULATION VIBROTACTILE**
VORRICHTUNG ZUR VIBROTAKTILEN STIMULATION
VIBROTACTILE STIMULATION DEVICE

(30) Priorité: 24.11.2014 FR 1461375
(43) Date de publication de la demande: 04.10.2017
(73) Titulaire: INSERM - Institut National de la Santé et de la Recherche Médicale, 75013 Paris (FR); Université de Rennes, 35042 Rennes (FR)
(72) Inventeur: HERNANDEZ, Alfredo, F-35510 Cesson-Sevigne (FR)
(74) Mandataire: Le Forestier, Eric
(86) Numéro de dépôt international: PCT/IB2015/059084
(87) Numéro de publication internationale: WO 2016/083999

(56) Documents cités:
- WO-A1-2007/141345
- US-A1- 2013 102 937
- US-A1- 2013 197 321
- US-A1- 2014 180 036
- US-A1- 2014 206 955
- US-B1- 6 265 978
- US-B1- 6 275 213

## Description

### Domaine de l'invention

La présente invention concerne d'une façon générale les dispositifs de stimulation vibrotactile ou kinesthésique.

### Etat de la technique

La stimulation vibrotactile ou kinesthésique est connue dans l'état de l'art et couramment utilisée depuis plus de 20 ans dans différents domaines d'application, notamment, pour l'assistance sensorielle.

Ainsi le document US 5 035 242 A vise un dispositif de stimulation à l'usage des malentendants, le document WO 2009082682 A1 propose un dispositif pour les personnes avec déficience visuelle.

La stimulation vibrotactile est encore utilisée pour le traitement des apnées du sommeil, notamment chez le nouveau-né prématuré (voir en particulier US 5 555 891 A ou WO2007141345 A1). Ce dernier document décrit également la possibilité d'intégrer une circuiterie d'ECG, avec des électrodes séparées.

Plusieurs principes de transduction sont utilisés dans les effecteurs vibro-tactiles ou kinesthésiques. Dans leur grande majorité, ils délivrent une stimulation mécanique vibratoire à partir de l'application d'un signal électrique de caractéristiques prédéfinies.

En général, la commande de la stimulation mécanique vibratoire est effectuée en réponse à une détection d'apnée, typiquement en déterminant le flux d'air respiratoire dans le nez et/ou la bouche du sujet grâce à une canule reliée au dispositif.

Ce principe de base peut être en soi suffisant pour faire cesser la phase d'apnée, mais on constate aujourd'hui que les caractéristiques de la stimulation gagneraient à tenir compte d'autres facteurs biologiques.

### Résumé de l'invention

La présente invention vise à proposer un dispositif de stimulation vibrotactile, notamment mais non exclusivement pour le traitement de l'apnée du sommeil, qui soit capable également d'estimer au moins un autre paramètre dont la prise en compte peut être utile pour ajuster la stimulation.

On propose ainsi selon l'invention un dispositif de stimulation vibrotactile tel que défini dans la revendication 1.

On pourra compléter ces caractéristiques avec les caractéristiques préférées mais optionnelles des revendications dépendantes.

### Brève description des dessins

D'autres aspects, buts et avantages de la présente invention apparaîtront mieux à la lecture de la description détaillée suivante d'une forme de réalisation préférée de celle-ci, donnée à titre d'exemple non limitatif et faire en référence aux dessins annexés, sur lesquels :
La Figure 1 est une vue de face schématique d'un dispositif de stimulation vibrotactile en deux unités destinées respectivement à se placer en arrière des oreilles d'un sujet, et des différents éléments constitutifs de ce dispositif,
La Figure 2 est une vue de face schématique de la tête d'un sujet équipé des deux parties et d'un organe de raccordement électrique,
La Figure 3 illustre un exemple de signaux recueillis avec le dispositif, et
Les Figures 4a et 4b sont des vues en coupe transversale des deux unités dotées d'un moyen de fixation et de protection.

### Description détaillée d'une forme de réalisation préférée

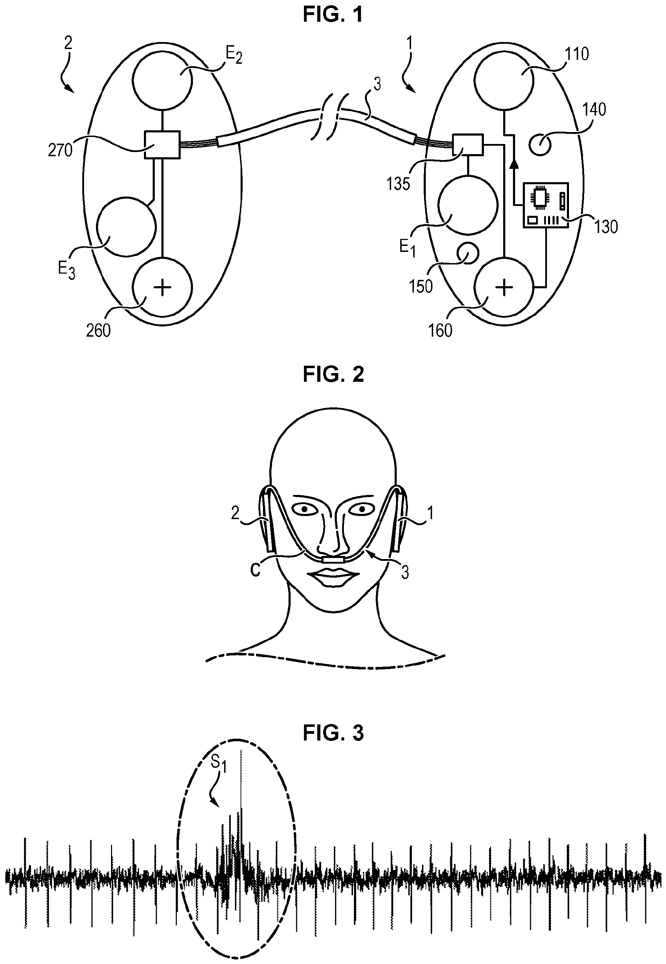

En référence aux Figures 1 et 2, un dispositif de stimulation vibrotactile ou kinesthésique 1 selon l'invention comprend deux unités 1 et 2, ici destinées à se placer derrière les deux oreilles d'un sujet.

Une première unité 1 comprend un effecteur ou excitateur électromécanique vibratoire 110. De façon préférée, l'effecteur 110 comprend un organe piézo-électrique ou un actuateur linéaire résonant. L'unité 1 comprend également des moyens de traitement numérique embarqués 130, typiquement sous la forme d'une carte électronique dotée d'un microcontrôleur, destinés à commander l'effecteur en fonction de certains paramètres déterminant en particulier (i) la présence d'un phénomène d'apnée du sommeil chez le sujet, (ii) l'évolution de la fréquence cardiaque et (iii) des caractéristiques extraites de l'électromyogramme (activité électrique musculaire) des muscles du cou.

En référence plus particulièrement à la Figure 2, le dispositif est apte à émettre des impulsions mécaniques d'excitation en fonction de critères tels que la détection d'un phénomène d'apnée du sommeil par des moyens et pouvant être conventionnels, et en particulier la détection d'un flux d'air respiratoire déterminée par une canule nasale C illustrée schématiquement sur la Figure 2.

Ces impulsions ont par exemple une fréquence de l'ordre de 100 à 400 Hz, et sont commandés par le circuit de commande 130 en réponse aux signaux de détection reçus via une interface filaire ou sans fil non illustrée.

L'unité 1 peut être dotée d'autres fonctionnalités. Elle peut par exemple intégrer un capteur de température 140 délivrant des signaux de température soit analogiques et convertis en signaux numériques au niveau du dispositif de traitement, soit directement numériques, un dispositif 150 de signalisation lumineuse (DEL) et/ou sonore (vibreur) exprimant l'état du dispositif, un interrupteur de marche/arrêt, etc.

Elle est alimentée par une pile bouton 160 ayant une capacité appropriée, ou éventuellement une batterie rechargeable, de façon filaire (par exemple sur le port USB D'un ordinateur) ou sans fil (par transmission inductive de puissance, de façon connue en soi pour les petits appareils électroniques).

Selon une variante de réalisation, les moyens de traitement numérique, ou une partie de ces moyens de traitement, peuvent être déportés dans un boîtier distinct du dispositif et soit porté par le patient, soit disposé à son voisinage, par exemple sur sa table de nuit, pendant le sommeil.

Des moyens de transmission sont alors prévus pour permettre la communication du dispositif avec le boîtier déporté, ces moyens pouvant être filaires ou sans fil pour un boîtier porté par le patient, et de préférence sans fil pour un boîtier fixe.

Selon une caractéristique de l'invention, le dispositif également est apte à détecter des phénomènes cardio-respiratoires exploitables par l'unité de commande 130 pour estimer les moments auxquels une stimulation à l'aide de l'effecteur 110 est nécessaire et pour déterminer les paramètres de cette stimulation.

A cet effet le dispositif comprend un ensemble d'électrodes destinées à être en contact avec la peau du patient lorsque les unités sont posées.

Une première électrode E1 est située dans l'unité 1, tandis que deux autres électrodes E2 et E3 sont situées dans une seconde unité désignée par la référence 2, destinée à être posée sur le sujet à distance de la première unité 1. Par exemple, la deuxième unité 2 peut être appliquée sur l'os mastoïdien derrière l'oreille droite du patient, lorsque l'unité 1 est posée sur l'os mastoïdien en arrière de son oreille gauche, ou inversement.

Les électrodes E2 et E3 de l'unité 2 sont reliées électriquement à l'unité 1 de manière qu'un circuit de traitement des signaux délivrés par les électrodes, désigné par la référence 135, puisse analyser ces signaux et délivrer une indication d'activité cardiaque et/ou respiratoire du sujet. Avantageusement, cette liaison électrique est réalisée à l'aide d'un ensemble de conducteurs 3 associés à une canule nasale C utilisée, de façon connue en soi, pour estimer un flux d'air de l'air au voisinage des narines et de la bouche du sujet et en déduire l'existence ou non d'une activité respiratoire du sujet.

Ces conducteurs peuvent être intégrés dans une paroi de la canule C, ou fixés à celle-ci de toute autre manière.

En variante, on peut prévoir de faire passer les conducteurs 3 en arrière de la tête du sujet, par exemple de façon intégrée à un bandeau élastique ou en étant retenus par un tel bandeau.

Lorsque les unités sont placées en arrière des deux oreilles comme illustré sur la figure 2, ces électrodes permettent de recueillir un mélange de signaux électrophysiologiques reflétant principalement l'activité électrique cardiaque et l'activité des muscles proches de la région mastoïdienne.

L'une des électrodes, par exemple l'électrode E3 située dans l'unité 2, est une électrode de référence, tandis que les signaux recueillis par les électrodes E1 et E2 distantes l'une de l'autre sont appliqués aux entrées d'un amplificateur différentiel à gain élevé prévu dans le circuit de traitement 135.

La Figure 3 donne un exemple de l'allure des signaux recueillis, qui reflètent le mélange d'activités précité. On observe des pics répartis de façon assez régulière, qui correspondent aux pulsations cardiaques (onde R de l'électrocardiogramme ECG). On observe également une activité électrique des muscles environnants (EMG) révélée par un signal de plus faible amplitude et de contenu fréquentiel plus élevé, se produisant sur toute l'étendue de l'échantillon représenté. Ce signal EMG est susceptible de cacher les ondes P et T de l'ECG. En particulier, la perturbation (zone de signal S1) illustrée sur la Figure 3, correspond à une augmentation significative de l'activité musculaire dans la région mastoïdienne, révélée par l'augmentation d'énergie du signal EMG.

Le circuit de traitement 135 est apte à séparer mutuellement ces signaux ECG et EMG, afin de pouvoir les traiter distinctement. Par exemple, il met un oeuvre un procédé de séparation de sources ou de filtrage adaptatif linéaire ou non-linéaire, de façon connue en soi.

Une fois les signaux séparés, le signal EMG résultant peut être utilisé pour estimer le niveau de tonus des muscles environnants. En particulier, il a été déterminé qu'une diminution du tonus des muscles du pharynx joue un rôle important dans la genèse des apnées obstructives. D'autre part, une reprise du tonus musculaire (comme celle révélée par la partie de signal S1 sur la Figure 3) est souvent associée à la fin d'une apnée obstructive. Le tonus musculaire ainsi estimé fournit également de l'information sur l'état de sommeil du patient, particulièrement pour différencier les états de veille et de sommeil.

Le traitement de signaux selon cet aspect de l'invention permet donc d'analyser ce tonus musculaire afin (i) d'activer la thérapie de stimulation de façon précoce, (ii), de différencier les apnées centrales des apnées obstructives, (iii) de piloter de façon optimale la stimulation et (iv) d'optimiser les paramètres de détection des événements respiratoires, par exemple, en fonction des stades de sommeil.

Concernant le signal ECG résultant de la séparation, la principale information extraite est la fréquence cardiaque, dont il s'avère qu'elle est fortement perturbée pendant les épisodes d'apnée, et qui est utilisée de façon connue en soi comme variable de contrôle permettant l'optimisation de la stimulation vibrotactile (voir WO2007141345 A1 déjà cité).

Le circuit 135 est fonctionnellement relié au circuit 130 de commande de la stimulation vibrotactile, de manière à pouvoir ajuster les stratégies de stimulation en fonction non seulement de l'activité respiratoire proprement dite, mesurée par la canule C et éventuellement par d'autres capteurs, mais également du rythme cardiaque du sujet ainsi que du tonus musculaire dans la région mastoïdienne, comme indiqué ci-dessus. On notera que les traitements de séparation des signaux issus des électrodes (notamment filtrage, décomposition fréquentielle, détection d'amplitude, etc.) peuvent être effectués soit dans le circuit 135, soit dans le circuit 130. Par ailleurs, l'homme du métier comprendra que les circuits 130 et 135 peuvent être fusionnés en un seul circuit.

Les conducteurs 3 comprennent de préférence au moins deux conducteurs, à savoir un pour chaque électrode E2 et E3.

On peut par ailleurs répartir les différentes fonctions du dispositif entre les deux unités 1 et 2, et notamment l'unité 2 peut également comporter un circuit de traitement ou de commande, une batterie, des dispositifs de signalisation, un commutateur marche/arrêt, etc.

Naturellement le nombre de conducteurs dans le faisceau 3 sera adapté par l'homme du métier en fonction de la répartition des fonctions entre les deux unités.

Par ailleurs, si l'espace dans l'unité 1 s'avère suffisant, on pourra y intégrer l'électrode de référence E3.

Les dimensions de chaque unité du dispositif sont typiquement de 3 à 8 cm selon le grand axe et de 2 à 6 cm selon le petit axe.

A noter également que l'unité 2 peut être disposé en tout emplacement autre que l'arrière de l'oreille opposée à celle où se trouve l'unité 1, étant observé que pour recueillir des signaux d'activité cardiaque et/ou musculaire appropriés, la distance entre les électrodes E1 et E2 doit être suffisamment grande (typiquement d'au moins 10 à 20 cm).

La nature de la liaison entre les deux unités 1 et 2 sera alors adaptée en conséquence.

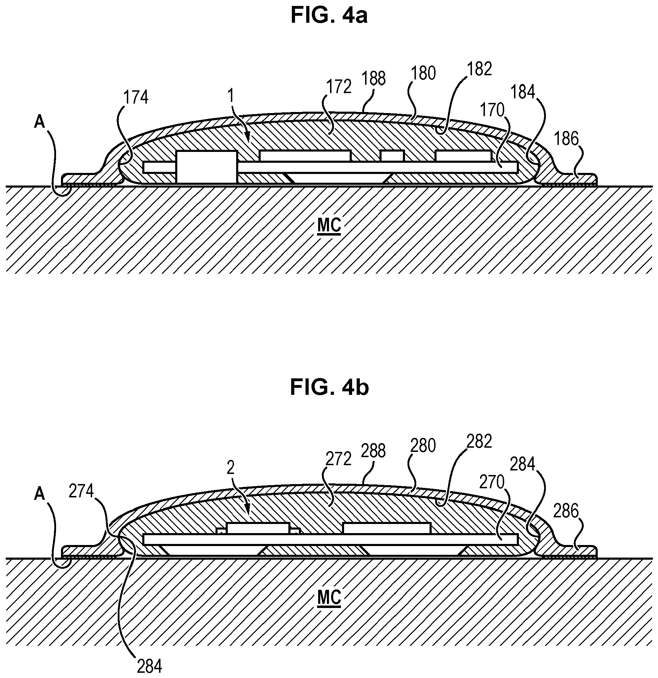

Les Figures 4a et 4b illustrent une implémentation pratique du dispositif selon l'invention. Les différents éléments de chaque unité 1, 2 sont montés sur une platine, respectivement 170, 270, l'ensemble étant enrobé dans un bloc ou coque, respectivement 172, 272, par exemple en résine, qui dispose de bords périphériques respectivement 174, 274 appropriés au couplage avec un moyen de fixation à la peau. Un tel moyen de fixation peut être une enveloppe jetable respectivement 180, 280, réalisée en un matériau élastique et capable de recevoir l'unité respective 1, 2 dans une cavité interne respective 182, 282, en retenant celui-ci par les bords respectifs 174, 274 du bloc de résine d'encapsulation, engagés dans une encoche périphérique respective 184, 284 de ladite enveloppe.

Un adhésif biocompatible A peut être prévu sur le bord périphérique respectif 186, 286 de chaque enveloppe, destiné à être en contact avec la peau, tandis qu'une paroi de dessus respectivement 188, 288 de l'enveloppe recouvre et étanchéifié entièrement le l'unité associée, encapsulée dans son bloc de résine respectif 172, 272.

On comprend que l'unité encapsulée 1 ou 2 peut être aisément extraite de son enveloppe 180, 280 pour la remplacer par une enveloppe neuve. L'adhésif A peut être couvert, de façon classique en soi, d'une pellicule de protection pelable à retirer avant usage.

Les unités formant le dispositif de stimulation selon l'invention peuvent être fixées sur tout site adapté (derrière les oreilles, sur les thorax latéraux, la plante des pieds, etc.), les enveloppes 180, 280 et leurs caractéristiques étant alors adaptées à l'usage prévu. De même, la liaison électrique entre les deux unités est adaptée en conséquence.

Les Figures 4a et 4b montrent que les électrodes sont directement en contact avec le milieu à stimuler MC, tandis que l'effecteur 10 peut appliquer les vibrations au milieu soit par contact direct, soit via le matériau de la coque 172, choisi alors pour assurer un couplage mécanique approprié avec le milieu MC.

On peut prévoir également, de façon connue en soi, que la surface des électrodes soit recouverte par un milieu favorisant la conduction électrique.

En particulier, on peut adjoindre au dispositif toute fonctionnalité indépendante ou corrélée à la stimulation vibratoire, et notamment, outre la mesure de température, toute détection ou captage d'un autre paramètre biologique.

## Revendications

1. Dispositif (1) de stimulation vibrotactile pour effectuer une stimulation corporelle dans la lutte contre les apnées du sommeil, destiné à être appliqué contre un milieu corporel à stimuler (MC), réalisé sous forme d'une première unité fonctionnelle (1) comprenant, intégrés à un premier boîtier, un effecteur vibrant (10) apte à appliquer audit milieu des impulsions d'énergie mécanique vibratoire propres à effectuer la stimulation corporelle, et des moyens (130) de commande de l'effecteur selon des règles de stimulation, la première unité fonctionnelle (1) abritant en outre, également de façon intégrée au premier boîtier, une première électrode (E1), le dispositif (1) comprenant également une deuxième unité (2) reliée à la première unité par un faisceau de conducteurs (3) et comprenant un deuxième boîtier intégrant au moins une deuxième électrode (E2, E3), les premier et deuxième boîtiers étant adaptés pour être appliqués sur le corps à distance l'un de l'autre, et les au moins deux électrodes étant aptes à coopérer ensemble pour fournir des signaux représentatifs d'une activité cardiaque et d'une activité musculaire sur le milieu à stimuler, lesdits moyens de commande (130) comprenant un circuit de traitement (135) apte à dériver des signaux fournis par les électrodes au moins une information d'activité cardiaque et une information d'activité musculaire dans la région des électrodes telle que la région mastoïdienne et étant aptes à commander l'effecteur (10) en fonction des informations d'activité délivrées par le circuit de traitement (135).

2. Dispositif selon la revendication 1, **caractérisé en ce que** la première unité (1) ou la deuxième unité (2) abrite une troisième électrode (E3) formant électrode de référence.

3. Dispositif selon la revendication 1 ou 2, comprenant en outre une canule nasale (C) pour la détection d'un flux d'air respiratoire, et **caractérisé en ce que** le faisceau de conducteurs (3) est associé à la canule nasale.

4. Dispositif selon l'une des revendications 1 à 3, **caractérisé en ce que** le circuit de traitement (135) est apte à réaliser une séparation entre des signaux d'électrocardiogramme et des signaux d'électromyogramme.

5. Dispositif selon l'une des revendications 1 à 4, **caractérisé en ce que** chaque unité (1 ; 2) comprend une platine (170 ; 270) portant ses différents organes, et une enveloppe souple jetable (180 ; 280) apte à recevoir la platine de façon amovible.

6. Dispositif selon la revendication 5, **caractérisé en ce que** l'ensemble des organes portés par chaque platine (170 ; 270) sont encapsulés.

7. Dispositif selon la revendication 5 ou 6, **caractérisé en ce que** chaque enveloppe souple (180 ; 280) comporte des aménagements (184 ; 284) aptes à recevoir et retenir élastiquement au moins une partie de bord (174 ; 274) de l'unité associée.

8. Dispositif selon l'une des revendications 5 à 7, **caractérisé en ce que** chaque enveloppe (180 ; 280) présente un bord périphérique (186 ; 286) pourvu d'un adhésif (A).

## Patentansprüche

1. Vorrichtung (1) zur vibrotaktilen Stimulation, um eine körperliche Stimulation im Kampf gegen die Schlafapnoe durchzuführen, die dazu vorgesehen ist, an einem zu stimulierenden Körpermedium (MC) angelegt zu werden, umgesetzt in Form einer ersten funktionellen Einheit (1), die in einem ersten Gehäuse integriert ein vibrierendes Durchführungselement (10), das geeignet ist, am Medium Impulse mechanischer Energie eigens zum Durchführen der körperlichen Stimulation anzulegen, und Regelungsmittel (130) des Durchführungselements nach den Regeln der Stimulation umfasst, wobei die erste funktionelle Einheit (1) weiter ebenfalls integriert im ersten Gehäuse eine erste Elektrode (E1) umfasst, wobei die Vorrichtung (1) ebenfalls eine zweite Einheit (2) umfasst, die durch ein Leiterbündel (3) mit der ersten Einheit verbunden ist, und ein zweites Gehäuse umfasst, das mindestens eine zweite Elektrode (E2, E3) integriert, wobei das erste und das zweite Gehäuse dazu angepasst sind, voneinander beabstandet am Körper angelegt zu werden, und die mindestens zwei Elektroden geeignet sind, zusammenzuwirken, um Signale bereitzustellen, die eine Herzaktivität und eine Muskelaktivität auf dem zu stimulierenden Medium darstellen, wobei die Regelungsmittel (130) eine Behandlungsschaltung (135) umfassen, die geeignet ist, aus den von den Elektroden bereitgestellten Signalen mindestens eine Information über eine Herzaktivität und eine Information über eine Muskelaktivität im Bereich der Elektroden, wie beispielsweise dem Mastoidbereich, abzuleiten und geeignet ist, das Durchführungsmittel (10) in Abhängigkeit von den von der Behandlungsschaltung (135) gelieferten Aktivitätsinformationen zu regeln.

2. Vorrichtung nach Anspruch 1, **dadurch gekennzeichnet, dass** die erste Einheit (1) oder die zweite Einheit (2) eine dritte Elektrode (E3) aufnimmt, die eine Referenzelektrode bildet.

3. Vorrichtung nach Anspruch 1 oder 2, weiter umfassend eine Nasenkanüle (C) zur Erfassung eines Atemzugs, und **dadurch gekennzeichnet, dass** das Leiterbündel (3) der Nasenkanüle zugeordnet ist.

4. Vorrichtung nach einem der Ansprüche 1 bis 3, **dadurch gekennzeichnet, dass** die Behandlungsschaltung (135) geeignet ist, eine Trennung zwischen Elektrodiagrammsignalen und Elektromyogrammsignalen durchzuführen.

5. Vorrichtung nach einem der Ansprüche 1 bis 4, **dadurch gekennzeichnet, dass** jede Einheit (1; 2) eine Platine (170; 270) umfasst, die ihre unterschiedlichen Organe trägt, und eine nachgiebige Einweghülle (180; 280), die geeignet ist, die Platine auf abnehmbare Weise aufzunehmen.

6. Vorrichtung nach Anspruch 5, **dadurch gekennzeichnet, dass** die Gesamtheit der von jeder Platine (170; 270) getragenen Organe verkapselt sind.

7. Vorrichtung nach Anspruch 5 oder 6, **dadurch gekennzeichnet, dass** jede nachgiebige Hülle (180; 280) Einrichtungen (184; 284) umfasst, die geeignet sind, mindestens einen Randteil (174, 274) der zugeordneten Einheit aufzunehmen und elastisch zurückzuhalten.

8. Vorrichtung nach einem der Ansprüche 5 bis 7, **dadurch gekennzeichnet, dass** jede Hülle (180; 280) einen Umfangsrand (186; 286) aufweist, der mit einem Haftmittel (A) versehen ist.

## Claims

1. A vibrotactile stimulation device (1) for performing a bodily stimulation in combatting sleep apnea, intented to be applied against a body medium (MC) to be stimulated, embodied as a first functional unit (1) comprising, integrated in a first casing, a vibrating effector (10) capable of applying to said medium pulses of mechanical vibrational energy for performing the body stimulation, and means (130) for controlling the effector according to stimulation rules, the first functional unit (1) further housing, also in an integrated manner in the first casing, a first electrode (E1), the device (1) further comprising a second unit (2) connected to the first unit by a bundle of conductors (3) and comprising a second casing integrating at least one second electrode (E2, E3), the first and second casings being adapted to be applied to the body at a distance from one another, and the at least two electrodes being capable of cooperating together to provide signals representative of a cardiac activity and a muscular activity on the medium being stimulated, said control means (130) comprising a processing circuit (135) capable of deriving from the signals provided by the electrodes, at least one cardiac activity information and a muscular activity information in the region of the electrodes such as the mastoid region, and being capable of controlling the effector (10) according to the activity information delivered by the processing circuit (135).

2. The device as claimed in claim 1, **characterized in that** the first unit (1) or the second unit (2) houses a third electrode (E3) forming a reference electrode.

3. The device as claimed in claim 1 or 2, further comprising a nasal cannula (C) for the detection of a respiratory air flow, and **characterized in that** the bundle of conductors (3) is associated with the nasal cannula.

4. The device as claimed in one of claims 1 to 3, **characterized in that** the processing circuit (135) is capable of performing a separation between electrocardiogram signals and electromyogram signals.

5. The device as claimed in one of claims 1 to 4, **characterized in that** each unit (1; 2) comprises a board (170; 270) carrying its various elements, and a disposable flexible envelope (180; 280) capable of receiving the board in removable manner.

6. The device as claimed in claim 5, **characterized in that** the set of elements carried by each board (170; 270) are encapsulated.

7. The device as claimed in claim 5 or 6, **characterized in that** each flexible casing (180; 280) comprises arrangements (184; 284) capable of receiving and resiliently retaining at least one edge portion (174; 274) of the associated unit.

8. The device as claimed in one of claims 5 to 7, **characterized in that** each casing (180; 280) has a peripheral edge (186; 286) provided with an adhesive (A).
